# EUROPEAN PATENT SPECIFICATION

(11) **EP 2 258 836 B1**
(45) Date of publication and mention of the grant of the patent: **04.05.2016**
(21) Application number: 10180231.2
(22) Date of filing: 07.09.2005
(51) Int. Cl.: C12N 9/28, B08B 7/00, B08B 17/02, C11D 3/386

(54) **Methods for preventing, removing, reducing, or disrupting biofilm**
Verfahren zur Vorbeugung, Entfernung, Verringerung oder Unterbrechung von Biofilm
Procédés permettant de détruire, de réduire, d'éliminer ou d'empêcher la formation d'un film biologique

(30) Priority: 10.09.2004 US 608535 P
(43) Date of publication of application: 08.12.2010
(62) Divisional of application: 05795624.5
(73) Proprietor: Novozymes North America, Inc., Franklinton, North Carolina 27525 (US); Novozymes A/S, 2880 Bagsvaerd (DK)
(72) Inventor: Deinhammer, Randy, Wake Forest, NC 27587 (US); Andersen, Carsten, 3500 Værløse (DK)
(74) Representative: Kofoed, Gertrud Sonne

(56) References cited:
- WO-A-01/98214
- WO-A-98/26807
- WO-A2-01/66712
- US-A1- 2004 018 607
- BOLTON DECLAN J ET AL: "Purification and characterization of the alpha-amylase of Bacillus flavothermus" ENZYME AND MICROBIAL TECHNOLOGY, vol. 20, no. 5, 1997, pages 340-343, XP002605725 ISSN: 0141-0229

### SEQUENCE LISTING

<110> Deinhammer, Randy Andersen, Carsten
<120> Methods for preventing or removing biofilms
<130> 10648
<160> 6
<170> PatentIn version 3.3
<210> 1
   <211> 1455
   <212> DNA
   <213> Bacillus sp.
<220>
   <221> CDS
   <222> (1)..(1455)
   <223> AA560
<220>
   <221> mat_peptide
   <222> (1)..()
<400> 1
   cac cat aat ggt acg aac ggc aca atg atg cag tac ttt gaa tgg tat      48
   His His Asn Gly Thr Asn Gly Thr Met Met Gln Tyr Phe Glu Trp Tyr
   1               5                   10                  15
   cta cca aat gac gga aac cat tgg aat aga tta agg tct gat gca agt      96
   Leu Pro Asn Asp Gly Asn His Trp Asn Arg Leu Arg Ser Asp Ala Ser
               20                  25                  30
   aac cta aaa gat aaa ggg atc tca gcg gtt tgg att cct cct gca tgg     144
   Asn Leu Lys Asp Lys Gly Ile Ser Ala Val Trp Ile Pro Pro Ala Trp
           35                  40                  45
   aag ggt gcc tct caa aat gat gtg ggg tat ggt gct tat gat ctg tat     192
   Lys Gly Ala Ser Gln Asn Asp Val Gly Tyr Gly Ala Tyr Asp Leu Tyr
       50                  55                  60
   gat tta gga gaa ttc aat caa aaa gga acc att cgt aca aaa tat gga     240
   Asp Leu Gly Glu Phe Asn Gln Lys Gly Thr Ile Arg Thr Lys Tyr Gly
   65                  70                  75                  80
   acg cgc aat cag tta caa gct gca gtt aac gcc ttg aaa agt aat gga     288
   Thr Arg Asn Gln Leu Gln Ala Ala Val Asn Ala Leu Lys Ser Asn Gly
                   85                  90                  95
   att caa gtg tat ggc gat gtt gta atg aat cat aaa ggg gga gca gac     336
   Ile Gln Val Tyr Gly Asp Val Val Met Asn His Lys Gly Gly Ala Asp
               100                 105                 110
   gct acc gaa atg gtt agg gca gtt gaa gta aac ccg aat aat aga aat      384
   Ala Thr Glu Met Val Arg Ala Val Glu Val Asn Pro Asn Asn Arg Asn
           115                 120                 125
   caa gaa gtg tcc ggt gaa tat aca att gag gct tgg aca aag ttt gac      432
   Gln Glu Val Ser Gly Glu Tyr Thr Ile Glu Ala Trp Thr Lys Phe Asp
       130                 135                 140
   ttt cca gga cga ggt aat act cat tca aac ttc aaa tgg aga tgg tat      480
   Phe Pro Gly Arg Gly Asn Thr His Ser Asn Phe Lys Trp Arg Trp Tyr
   145                 150                 155                 160
   cac ttt gat gga gta gat tgg gat cag tca cgt aag ctg aac aat cga      528
   His Phe Asp Gly Val Asp Trp Asp Gln Ser Arg Lys Leu Asn Asn Arg
                   165                 170                 175
   att tat aaa ttt aga ggt gat gga aaa ggg tgg gat tgg gaa gtc gat      576
   Ile Tyr Lys Phe Arg Gly Asp Gly Lys Gly Trp Asp Trp Glu Val Asp
               180                 185                 190
   aca gaa aac ggt aac tat gat tac cta atg tat gca gat att gac atg      624
   Thr Glu Asn Gly Asn Tyr Asp Tyr Leu Met Tyr Ala Asp Ile Asp Met
           195                 200                 205
   gat cac cca gag gta gtg aat gag cta aga aat tgg ggt gtt tgg tat      672
   Asp His Pro Glu Val Val Asn Glu Leu Arg Asn Trp Gly Val Trp Tyr
       210                 215                 220
   acg aat aca tta ggc ctt gat ggt ttt aga ata gat gca gta aaa cat      720
   Thr Asn Thr Leu Gly Leu Asp Gly Phe Arg Ile Asp Ala Val Lys His
   225                 230                 235                 240
   ata aaa tac agc ttt act cgt gat tgg att aat cat gtt aga agt gca      768
   Ile Lys Tyr Ser Phe Thr Arg Asp Trp Ile Asn His Val Arg Ser Ala
                   245                 250                 255
   act ggc aaa aat atg ttt gcg gtt gcg gaa ttt tgg aaa aat gat tta      816
   Thr Gly Lys Asn Met Phe Ala Val Ala Glu Phe Trp Lys Asn Asp Leu
               260                 265                 270
   ggt gct att gaa aac tat tta aac aaa aca aac tgg aac cat tca gtc      864
   Gly Ala Ile Glu Asn Tyr Leu Asn Lys Thr Asn Trp Asn His Ser Val
           275                 280                 285
   ttt gat gtt ccg ctg cac tat aac ctc tat aat gct tca aaa agc gga      912
   Phe Asp Val Pro Leu His Tyr Asn Leu Tyr Asn Ala Ser Lys Ser Gly
       290                 295                 300
   ggg aat tat gat atg agg caa ata ttt aat ggt aca gtc gtg caa aga      960
   Gly Asn Tyr Asp Met Arg Gln Ile Phe Asn Gly Thr Val Val Gln Arg
   305                 310                 315                 320
   cat cca atg cat gct gtt aca ttt gtt gat aat cat gat tcg caa cct     1008
   His Pro Met His Ala Val Thr Phe Val Asp Asn His Asp Ser Gln Pro
                   325                 330                 335
   gaa gaa gct tta gag tct ttt gtt gaa gaa tgg ttc aaa cca tta gcg     1056
   Glu Glu Ala Leu Glu Ser Phe Val Glu Glu Trp Phe Lys Pro Leu Ala
               340                 345                 350
   tat gct ttg aca tta aca cgt gaa caa ggc tac cct tct gta ttt tat     1104
   Tyr Ala Leu Thr Leu Thr Arg Glu Gln Gly Tyr Pro Ser Val Phe Tyr
           355                 360                 365
   gga gat tat tat ggc att cca acg cat ggt gta cca gcg atg aaa tcg     1152
   Gly Asp Tyr Tyr Gly Ile Pro Thr His Gly Val Pro Ala Met Lys Ser
       370                 375                 380
   aaa att gac ccg att cta gaa gcg cgt caa aag tat gca tat gga aga     1200
   Lys Ile Asp Pro Ile Leu Glu Ala Arg Gln Lys Tyr Ala Tyr Gly Arg
   385                 390                 395                 400
   caa aat gac tac tta gac cat cat aat atc atc ggt tgg aca cgt gaa     1248
   Gln Asn Asp Tyr Leu Asp His His Asn Ile Ile Gly Trp Thr Arg Glu
                   405                 410                 415
   ggg aat aca gca cac ccc aac tcc ggt tta gct act atc atg tcc gat     1296
   Gly Asn Thr Ala His Pro Asn Ser Gly Leu Ala Thr Ile Met Ser Asp
               420                 425                 430
   ggg gca gga gga aat aag tgg atg ttt gtt ggg cgt aat aaa gct ggt     1344
   Gly Ala Gly Gly Asn Lys Trp Met Phe Val Gly Arg Asn Lys Ala Gly
           435                 440                 445
   caa gtt tgg acc gat atc act gga aat cgt gca ggt act gtt acg att     1392
   Gln Val Trp Thr Asp Ile Thr Gly Asn Arg Ala Gly Thr Val Thr Ile
       450                 455                 460
   aat gct gat gga tgg ggt aat ttt tct gta aat gga gga tca gtt tct     1440
   Asn Ala Asp Gly Trp Gly Asn Phe Ser Val Asn Gly Gly Ser Val Ser
   465                 470                 475                 480
   att tgg gta aac aaa                                                 1455
   Ile Trp Val Asn Lys
                   485
<210> 2
   <211> 485
   <212> PRT
   <213> Bacillus sp.
<400> 2
   His His Asn Gly Thr Asn Gly Thr Met Met Gln Tyr Phe Glu Trp Tyr
   1               5                   10                  15
   Leu Pro Asn Asp Gly Asn His Trp Asn Arg Leu Arg Ser Asp Ala Ser
               20                  25                  30
   Asn Leu Lys Asp Lys Gly Ile Ser Ala Val Trp Ile Pro Pro Ala Trp
           35                  40                  45
   Lys Gly Ala Ser Gln Asn Asp Val Gly Tyr Gly Ala Tyr Asp Leu Tyr
       50                  55                  60
   Asp Leu Gly Glu Phe Asn Gln Lys Gly Thr Ile Arg Thr Lys Tyr Gly
   65                  70                  75                  80
   Thr Arg Asn Gln Leu Gln Ala Ala Val Asn Ala Leu Lys Ser Asn Gly
                   85                  90                  95
   Ile Gln Val Tyr Gly Asp Val Val Met Asn His Lys Gly Gly Ala Asp
               100                 105                 110
   Ala Thr Glu Met Val Arg Ala Val Glu Val Asn Pro Asn Asn Arg Asn
           115                 120                 125
   Gln Glu Val Ser Gly Glu Tyr Thr Ile Glu Ala Trp Thr Lys Phe Asp
       130                 135                 140
   Phe Pro Gly Arg Gly Asn Thr His Ser Asn Phe Lys Trp Arg Trp Tyr
   145                 150                 155                 160
   His Phe Asp Gly Val Asp Trp Asp Gln Ser Arg Lys Leu Asn Asn Arg
                   165                 170                 175
   Ile Tyr Lys Phe Arg Gly Asp Gly Lys Gly Trp Asp Trp Glu Val Asp
               180                 185                 190
   Thr Glu Asn Gly Asn Tyr Asp Tyr Leu Met Tyr Ala Asp Ile Asp Met
           195                 200                 205
   Asp His Pro Glu Val Val Asn Glu Leu Arg Asn Trp Gly Val Trp Tyr
       210                 215                 220
   Thr Asn Thr Leu Gly Leu Asp Gly Phe Arg Ile Asp Ala Val Lys His
   225                 230                 235                 240
   Ile Lys Tyr Ser Phe Thr Arg Asp Trp Ile Asn His Val Arg Ser Ala
                   245                 250                 255
   Thr Gly Lys Asn Met Phe Ala Val Ala Glu Phe Trp Lys Asn Asp Leu
               260                 265                 270
   Gly Ala Ile Glu Asn Tyr Leu Asn Lys Thr Asn Trp Asn His Ser Val
           275                 280                 285
   Phe Asp Val Pro Leu His Tyr Asn Leu Tyr Asn Ala Ser Lys Ser Gly
       290                 295                 300
   Gly Asn Tyr Asp Met Arg Gln Ile Phe Asn Gly Thr Val Val Gln Arg
   305                 310                 315                 320
   His Pro Met His Ala Val Thr Phe Val Asp Asn His Asp Ser Gln Pro
                   325                 330                 335
   Glu Glu Ala Leu Glu Ser Phe Val Glu Glu Trp Phe Lys Pro Leu Ala
               340                 345                 350
   Tyr Ala Leu Thr Leu Thr Arg Glu Gln Gly Tyr Pro Ser Val Phe Tyr
           355                 360                 365
   Gly Asp Tyr Tyr Gly Ile Pro Thr His Gly Val Pro Ala Met Lys Ser
       370                 375                 380
   Lys Ile Asp Pro Ile Leu Glu Ala Arg Gln Lys Tyr Ala Tyr Gly Arg
   385                 390                 395                 400
   Gln Asn Asp Tyr Leu Asp His His Asn Ile Ile Gly Trp Thr Arg Glu
                   405                 410                 415
   Gly Asn Thr Ala His Pro Asn Ser Gly Leu Ala Thr Ile Met Ser Asp
               420                 425                 430
   Gly Ala Gly Gly Asn Lys Trp Met Phe Val Gly Arg Asn Lys Ala Gly
           435                 440                 445
   Gln Val Trp Thr Asp Ile Thr Gly Asn Arg Ala Gly Thr Val Thr Ile
       450                 455                 460
   Asn Ala Asp Gly Trp Gly Asn Phe Ser Val Asn Gly Gly Ser Val Ser
   465                 470                 475                 480
   Ile Trp Val Asn Lys
                   485
<210> 3
   <211> 1860
   <212> DNA
   <213> Bacillus flavothermus
<220>
   <221> CDS
   <222> (1)..(1857)
<220>
   <221> sig_peptide
   <222> (1)..(99)
<220>
   <221> mat_peptide
   <222> (100)..(1857)
<220>
   <221> misc_feature
   <222> (100)..(1551)
   <223> catalytic domain
<220>
   <221> misc_feature
   <222> (1552)..(1857)
   <223> carbohydrate binding domain
<400> 3
   atg tcc cta ttc aaa aaa agc ttt ccg tgg att tta tcc cta ctt ctt       48
   Met Ser Leu Phe Lys Lys Ser Phe Pro Trp Ile Leu Ser Leu Leu Leu
               -30                 -25                 -20
   ttg ttt tcg ttt att gct cct ttt tcc att caa aca gaa aaa gtc cga       96
   Leu Phe Ser Phe Ile Ala Pro Phe Ser Ile Gln Thr Glu Lys Val Arg
           -15                 -10                 -5
   gct gga agt gtg ccg gta aat ggc aca atg atg caa tat ttc gaa tgg      144
   Ala Gly Ser Val Pro Val Asn Gly Thr Met Met Gln Tyr Phe Glu Trp
   -1  1               5                   10                  15
   tac ctt cca gac gat gga aca cta tgg acg aaa gta gca aat aac gct      192
   Tyr Leu Pro Asp Asp Gly Thr Leu Trp Thr Lys Val Ala Asn Asn Ala
                   20                  25                  30
   caa tct tta gcg aat ctt ggc att act gcc ctt tgg ctt ccc cct gcc      240
   Gln Ser Leu Ala Asn Leu Gly Ile Thr Ala Leu Trp Leu Pro Pro Ala
               35                  40                  45
   tat aaa gga aca agc agc agt gac gtt gga tat ggc gtt tat gat tta      288
   Tyr Lys Gly Thr Ser Ser Ser Asp Val Gly Tyr Gly Val Tyr Asp Leu
           50                  55                  60
   tat gac ctt gga gag ttt aat caa aaa gga act gtc cga aca aaa tac      336
   Tyr Asp Leu Gly Glu Phe Asn Gln Lys Gly Thr Val Arg Thr Lys Tyr
       65                  70                  75
   ggg aca aaa aca caa tat atc caa gca atc caa gcg gcg cat aca gca      384
   Gly Thr Lys Thr Gln Tyr Ile Gln Ala Ile Gln Ala Ala His Thr Ala
   80                  85                  90                  95
   ggg atg caa gta tat gca gat gtc gtc ttt aac cat aaa gcc ggt gca      432
   Gly Met Gln Val Tyr Ala Asp Val Val Phe Asn His Lys Ala Gly Ala
                   100                 105                 110
   gat gga aca gaa cta gtc gat gca gta gaa gta aat cct tct gac cgc      480
   Asp Gly Thr Glu Leu Val Asp Ala Val Glu Val Asn Pro Ser Asp Arg
               115                 120                 125
   aat caa gaa ata tca gga aca tat caa atc caa gcg tgg aca aaa ttt      528
   Asn Gln Glu Ile Ser Gly Thr Tyr Gln Ile Gln Ala Trp Thr Lys Phe
           130                 135                 140
   gat ttt cct ggt cgt gga aac acc tat tct agt ttt aaa tgg cgt tgg      576
   Asp Phe Pro Gly Arg Gly Asn Thr Tyr Ser Ser Phe Lys Trp Arg Trp
       145                 150                 155
   tat cat ttc gat gga acg gac tgg gat gag agt aga aaa cta aat cgt      624
   Tyr His Phe Asp Gly Thr Asp Trp Asp Glu Ser Arg Lys Leu Asn Arg
   160                 165                 170                 175
   att tac aag ttc cgc ggc acg gga aaa gca tgg gat tgg gaa gta gat      672
   Ile Tyr Lys Phe Arg Gly Thr Gly Lys Ala Trp Asp Trp Glu Val Asp
                   180                 185                 190
   aca gaa aac ggg aat tat gac tat ctc atg tat gca gat tta gat atg      720
   Thr Glu Asn Gly Asn Tyr Asp Tyr Leu Met Tyr Ala Asp Leu Asp Met
               195                 200                 205
   gat cat cca gag gtt gta tcc gaa cta aaa aat tgg gga aag tgg tat      768
   Asp His Pro Glu Val Val Ser Glu Leu Lys Asn Trp Gly Lys Trp Tyr
           210                 215                 220
   gta acc aca acc aat atc gac gga ttc cgt ctg gat gca gtg aag cat      816
   Val Thr Thr Thr Asn Ile Asp Gly Phe Arg Leu Asp Ala Val Lys His
       225                 230                 235
   att aaa tat agc ttt ttc ccg gac tgg cta tcg tac gta cga acc caa      864
   Ile Lys Tyr Ser Phe Phe Pro Asp Trp Leu Ser Tyr Val Arg Thr Gln
   240                 245                 250                 255
   aca caa aag cct ctt ttt gcc gtt ggg gaa ttt tgg agc tat gac att      912
   Thr Gln Lys Pro Leu Phe Ala Val Gly Glu Phe Trp Ser Tyr Asp Ile
                   260                 265                 270
   agc aag ttg cac aac tat att aca aag acg aac ggc tct atg tcc cta      960
   Ser Lys Leu His Asn Tyr Ile Thr Lys Thr Asn Gly Ser Met Ser Leu
               275                 280                 285
   ttc gat gcc ccg ctg cat aac aat ttt tat ata gca tcg aaa tca ggc      1008
   Phe Asp Ala Pro Leu His Asn Asn Phe Tyr Ile Ala Ser Lys Ser Gly
           290                 295                 300
   ggt tat ttt gat atg cgc aca tta ctc aac aac aca ttg atg aaa gat      1056
   Gly Tyr Phe Asp Met Arg Thr Leu Leu Asn Asn Thr Leu Met Lys Asp
       305                 310                 315
   cag cct aca tta gca gtc aca tta gtg gat aat cac gat act gag cca      1104
   Gln Pro Thr Leu Ala Val Thr Leu Val Asp Asn His Asp Thr Glu Pro
   320                 325                 330                 335
   ggg caa tct ctg cag tca tgg gtc gag cca tgg ttt aaa ccg tta gct      1152
   Gly Gln Ser Leu Gln Ser Trp Val Glu Pro Trp Phe Lys Pro Leu Ala
                   340                 345                 350
   tac gca ttt atc ttg acc cgc caa gaa ggt tat cct tgc gtc ttt tat      1200
   Tyr Ala Phe Ile Leu Thr Arg Gln Glu Gly Tyr Pro Cys Val Phe Tyr
               355                 360                 365
   gga gat tac tat ggt att cca aaa tac aac att cct gcg ctg aaa agc      1248
   Gly Asp Tyr Tyr Gly Ile Pro Lys Tyr Asn Ile Pro Ala Leu Lys Ser
           370                 375                 380
   aaa ctt gat ccg ctg tta att gcc aga aga gat tat gcc tat gga aca      1296
   Lys Leu Asp Pro Leu Leu Ile Ala Arg Arg Asp Tyr Ala Tyr Gly Thr
       385                 390                 395
   cag cac gac tat att gac agt gcg gat att atc ggt tgg acg cgg gaa      1344
   Gln His Asp Tyr Ile Asp Ser Ala Asp Ile Ile Gly Trp Thr Arg Glu
   400                 405                 410                 415
   gga gtg gct gaa aaa gca aat tca gga ctg gct gca ctc att acc gac      1392
   Gly Val Ala Glu Lys Ala Asn Ser Gly Leu Ala Ala Leu Ile Thr Asp
                   420                 425                 430
   ggg cct ggc gga agc aaa tgg atg tat gtt gga aaa caa cac gct ggc      1440
   Gly Pro Gly Gly Ser Lys Trp Met Tyr Val Gly Lys Gln His Ala Gly
               435                 440                 445
   aaa acg ttt tat gat tta acc ggc aat cga agt gat aca gtg aca atc      1488
   Lys Thr Phe Tyr Asp Leu Thr Gly Asn Arg Ser Asp Thr Val Thr Ile
           450                 455                 460
   aat gct gat gga tgg gga gaa ttt aaa gtc aat gga ggg tct gta tcc      1536
   Asn Ala Asp Gly Trp Gly Glu Phe Lys Val Asn Gly Gly Ser Val Ser
       465                 470                 475
   ata tgg gtt cca aaa ata tca acc act tcc caa ata aca ttt act gta      1584
   Ile Trp Val Pro Lys Ile Ser Thr Thr Ser Gln Ile Thr Phe Thr Val
   480                 485                 490                 495
   aat aac gcc aca acc gtt tgg gga caa aat gta tac gtt gtc ggg aat      1632
   Asn Asn Ala Thr Thr Val Trp Gly Gln Asn Val Tyr Val Val Gly Asn
                   500                 505                 510
   att tcg cag ctg ggg aac tgg gat cca gtc cac gca gtt caa atg acg     1680
   Ile Ser Gln Leu Gly Asn Trp Asp Pro Val His Ala Val Gln Met Thr
               515                 520                 525
   ccg tct tct tat cca aca tgg act gta aca atc cct ctt ctt caa ggg     1728
   Pro Ser Ser Tyr Pro Thr Trp Thr Val Thr Ile Pro Leu Leu Gln Gly
           530                 535                 540
   caa aac ata caa ttt aaa ttt atc aaa aaa gat tca gct gga aat gtc     1776
   Gln Asn Ile Gln Phe Lys Phe Ile Lys Lys Asp Ser Ala Gly Asn Val
       545                 550                 555
   att tgg gaa gat ata tcg aat cga aca tac acc gtc cca act gct gca     1824
   Ile Trp Glu Asp Ile Ser Asn Arg Thr Tyr Thr Val Pro Thr Ala Ala
   560                 565                 570                 575
   tcc gga gca tat aca gcc agc tgg aac gtg ccc tag                     1860
   Ser Gly Ala Tyr Thr Ala Ser Trp Asn Val Pro
                   580                 585
<210> 4
   <211> 619
   <212> PRT
   <213> Bacillus flavothermus
<400> 4
   Met Ser Leu Phe Lys Lys Ser Phe Pro Trp Ile Leu Ser Leu Leu Leu
               -30                 -25                 -20
   Leu Phe Ser Phe Ile Ala Pro Phe Ser Ile Gln Thr Glu Lys Val Arg
           -15                 -10                 -5
   Ala Gly Ser Val Pro Val Asn Gly Thr Met Met Gln Tyr Phe Glu Trp
   -1  1               5                   10                  15
   Tyr Leu Pro Asp Asp Gly Thr Leu Trp Thr Lys Val Ala Asn Asn Ala
                   20                  25                  30
   Gln Ser Leu Ala Asn Leu Gly Ile Thr Ala Leu Trp Leu Pro Pro Ala
               35                  40                  45
   Tyr Lys Gly Thr Ser Ser Ser Asp Val Gly Tyr Gly Val Tyr Asp Leu
           50                  55                  60
   Tyr Asp Leu Gly Glu Phe Asn Gln Lys Gly Thr Val Arg Thr Lys Tyr
       65                  70                  75
   Gly Thr Lys Thr Gln Tyr Ile Gln Ala Ile Gln Ala Ala His Thr Ala
   80                  85                  90                  95
   Gly Met Gln Val Tyr Ala Asp Val Val Phe Asn His Lys Ala Gly Ala
                   100                 105                 110
   Asp Gly Thr Glu Leu Val Asp Ala Val Glu Val Asn Pro Ser Asp Arg
               115                 120                 125
   Asn Gln Glu Ile Ser Gly Thr Tyr Gln Ile Gln Ala Trp Thr Lys Phe
           130                 135                 140
   Asp Phe Pro Gly Arg Gly Asn Thr Tyr Ser Ser Phe Lys Trp Arg Trp
       145                 150                 155
   Tyr His Phe Asp Gly Thr Asp Trp Asp Glu Ser Arg Lys Leu Asn Arg
   160                 165                 170                 175
   Ile Tyr Lys Phe Arg Gly Thr Gly Lys Ala Trp Asp Trp Glu Val Asp
                   180                 185                 190
   Thr Glu Asn Gly Asn Tyr Asp Tyr Leu Met Tyr Ala Asp Leu Asp Met
               195                 200                 205
   Asp His Pro Glu Val Val Ser Glu Leu Lys Asn Trp Gly Lys Trp Tyr
           210                 215                 220
   Val Thr Thr Thr Asn Ile Asp Gly Phe Arg Leu Asp Ala Val Lys His
       225                 230                 235
   Ile Lys Tyr Ser Phe Phe Pro Asp Trp Leu Ser Tyr Val Arg Thr Gln
   240                 245                 250                 255
   Thr Gln Lys Pro Leu Phe Ala Val Gly Glu Phe Trp Ser Tyr Asp Ile
                   260                 265                 270
   Ser Lys Leu His Asn Tyr Ile Thr Lys Thr Asn Gly Ser Met Ser Leu
               275                 280                 285
   Phe Asp Ala Pro Leu His Asn Asn Phe Tyr Ile Ala Ser Lys Ser Gly
           290                 295                 300
   Gly Tyr Phe Asp Met Arg Thr Leu Leu Asn Asn Thr Leu Met Lys Asp
       305                 310                 315
   Gln Pro Thr Leu Ala Val Thr Leu Val Asp Asn His Asp Thr Glu Pro
   320                 325                 330                 335
   Gly Gln Ser Leu Gln Ser Trp Val Glu Pro Trp Phe Lys Pro Leu Ala
                   340                 345                 350
   Tyr Ala Phe Ile Leu Thr Arg Gln Glu Gly Tyr Pro Cys Val Phe Tyr
               355                 360                 365
   Gly Asp Tyr Tyr Gly Ile Pro Lys Tyr Asn Ile Pro Ala Leu Lys Ser
           370                 375                 380
   Lys Leu Asp Pro Leu Leu Ile Ala Arg Arg Asp Tyr Ala Tyr Gly Thr
       385                 390                 395
   Gln His Asp Tyr Ile Asp Ser Ala Asp Ile Ile Gly Trp Thr Arg Glu
   400                 405                 410                 415
   Gly Val Ala Glu Lys Ala Asn Ser Gly Leu Ala Ala Leu Ile Thr Asp
                   420                 425                 430
   Gly Pro Gly Gly Ser Lys Trp Met Tyr Val Gly Lys Gln His Ala Gly
               435                 440                 445
   Lys Thr Phe Tyr Asp Leu Thr Gly Asn Arg Ser Asp Thr Val Thr Ile
           450                 455                 460
   Asn Ala Asp Gly Trp Gly Glu Phe Lys Val Asn Gly Gly Ser Val Ser
       465                 470                 475
   Ile Trp Val Pro Lys Ile Ser Thr Thr Ser Gln Ile Thr Phe Thr Val
   480                 485                 490                 495
   Asn Asn Ala Thr Thr Val Trp Gly Gln Asn Val Tyr Val Val Gly Asn
                   500                 505                 510
   Ile Ser Gln Leu Gly Asn Trp Asp Pro Val His Ala Val Gln Met Thr
               515                 520                 525
   Pro Ser Ser Tyr Pro Thr Trp Thr Val Thr Ile Pro Leu Leu Gln Gly
           530                 535                 540
   Gln Asn Ile Gln Phe Lys Phe Ile Lys Lys Asp Ser Ala Gly Asn Val
       545                 550                 555
   Ile Trp Glu Asp Ile Ser Asn Arg Thr Tyr Thr Val Pro Thr Ala Ala
   560                 565                 570                 575
   Ser Gly Ala Tyr Thr Ala Ser Trp Asn Val Pro
                   580                 585
<210> 5
   <211> 1920
   <212> DNA
   <213> Bacillus licheniformis
<220>
   <221> CDS
   <222> (421)..(1872)
<400> 5
   cggaagattg gaagtacaaa aataagcaaa agattgtcaa tcatgtcatg agccatgcgg     60
   gagacggaaa aatcgtctta atgcacgata tttatgcaac gttcgcagat gctgctgaag    120
   agattattaa aaagctgaaa gcaaaaggct atcaattggt aactgtatct cagcttgaag    180
   aagtgaagaa gcagagaggc tattgaataa atgagtagaa gcgccatatc ggcgcttttc    240
   ttttggaaga aaatataggg aaaatggtac ttgttaaaaa ttcggaatat ttatacaaca    300
   tcatatgttt cacattgaaa ggggaggaga atcatgaaac aacaaaaacg gctttacgcc    360
   cgattgctga cgctgttatt tgcgctcatc ttcttgctgc ctcattctgc agcagcggcg    420
   gca aat ctt aat ggg acg ctg atg cag tat ttt gaa tgg tac atg ccc      468
   aat gac ggc caa cat tgg agg cgt ttg caa aac gac tcg gca tat ttg      516
   gct gaa cac ggt att act gcc gtc tgg att ccc ccg gca tat aag gga      564
   acg agc caa gcg gat gtg ggc tac ggt gct tac gac ctt tat gat tta      612
   ggg gag ttt cat caa aaa ggg acg gtt cgg aca aag tac ggc aca aaa      660
   gga gag ctg caa tct gcg atc aaa agt ctt cat tcc cgc gac att aac      708
   gtt tac ggg gat gtg gtc atc aac cac aaa ggc ggc gct gat gcg acc      756
   gaa gat gta acc gcg gtt gaa gtc gat ccc gct gac cgc aac cgc gta      804
   att tca gga gaa cac cta att aaa gcc tgg aca cat ttt cat ttt ccg      852
   ggg cgc ggc agc aca tac agc gat ttt aaa tgg cat tgg tac cat ttt      900
   gac gga acc gat tgg gac gag tcc cga aag ctg aac cgc atc tat aag      948
   ttt caa gga aag gct tgg gat tgg gaa gtt tcc aat gaa aac ggc aac      996
   tat gat tat ttg atg tat gcc gac atc gat tat gac cat cct gat gtc     1044
   gca gca gaa att aag aga tgg ggc act tgg tat gcc aat gaa ctg caa     1092
   ttg gac ggt ttc cgt ctt gat gct gtc aaa cac att aaa ttt tct ttt     1140
   ttg cgg gat tgg gtt aat cat gtc agg gaa aaa acg ggg aag gaa atg     1188
   ttt acg gta gct gaa tat tgg cag aat gac ttg ggc gcg ctg gaa aac     1236
   tat ttg aac aaa aca aat ttt aat cat tca gtg ttt gac gtg ccg ctt     1284
   cat tat cag ttc cat gct gca tcg aca cag gga ggc ggc tat gat atg      1332
   agg aaa ttg ctg aac ggt acg gtc gtt tcc aag cat ccg ttg aaa tcg      1380
   gtt aca ttt gtc gat aac cat gat aca cag ccg ggg caa tcg ctt gag      1428
   tcg act gtc caa aca tgg ttt aag ccg ctt gct tac gct ttt att ctc      1476
   aca agg gaa tct gga tac cct cag gtt ttc tac ggg gat atg tac ggg      1524
   acg aaa gga gac tcc cag cgc gaa att cct gcc ttg aaa cac aaa att      1572
   gaa ccg atc tta aaa gcg aga aaa cag tat gcg tac gga gca cag cat      1620
   gat tat ttc gac cac cat gac att gtc ggc tgg aca agg gaa ggc gac      1668
   agc tcg gtt gca aat tca ggt ttg gcg gca tta ata aca gac gga ccc      1716
   ggt ggg gca aag cga atg tat gtc ggc cgg caa aac gcc ggt gag aca      1764
   tgg cat gac att acc gga aac cgt tcg gag ccg gtt gtc atc aat tcg      1812
   gaa ggc tgg gga gag ttt cac gta aac ggc ggg tcg gtt tca att tat      1860
   gtt caa aga tag aagagcagag aggacggatt tcctgaagga aatccgtttt          1912
   tttatttt                                                             1920
<210> 6
   <211> 483
   <212> PRT
   <213> Bacillus licheniformis
<400> 6

## Description

### Field of the Invention

The present invention relates to improved methods of preventing, removing, reducing, or disrupting biofilm formation on a surface.

### Description of the Related Art

Biofilms are biological films that develop and persist at the surfaces of biotic or abiotic objects in aqueous environments from the adsorption of microbial cells onto the solid surfaces. This adsorption can provide a competitive advantage for the microorganisms since they can reproduce, are accessible to a wider variety of nutrients and oxygen conditions, are not washed away, and are less sensitive to antimicrobial agents. The formation of the biofilm is also accompanied by the production of exo-polymeric materials (polysaccharides, polyuronic acids, alginates, glycoproteins, and proteins) which together with the cells form thick layers of differentiated structures separated by water-filled spaces. The resident microorganisms may be individual species of microbial cells or mixed communities of microbial cells, which may include aerobic and anaerobic bacteria, algae, protozoa, and fungi. Thus, the biofilm is a complex assembly of living microorganisms embedded in an organic structure composed of one or more matrix polymers which are secreted by the resident microorganisms.

Biofilms can develop into macroscopic structures several millimeters or centimeters in thickness and cover large surface areas. These formations can play a role in restricting or entirely blocking flow in plumbing systems, decreasing heat transfer in heat exchangers, or causing pathogenic problems in municipal water supplies, food processing, medical devices (e.g., catheters, orthopedic devices, implants, endoscopes). Moreover, biofilms often decrease the life of materials through corrosive action mediated by the embedded microorganisms. This biological fouling is a serious economic problem in industrial water process systems, pulp and paper production processes, cooling water systems, injection wells for oil recovery, cooling towers, porous media (sand and soil), marine environments, and air conditioning systems, and any closed water recirculation system. Biofilms are also a severe problem in medical science and industry causing dental plaque, infections (Costerton et al., 1999, Science 284: 1318-1322), contaminated endoscopes and contact lenses, prosthetic device colonisation and biofilm formation on medical implants.

The removal or prevention of biofilm traditionally requires the use of dispersants, surfactants, detergents, enzyme formulations, anti-microbials, biocides, boil-out procedures, and/or corrosive chemicals, *e.g.,* base. Procedures for using these measures are well known in the art. For example, removal of biofilm built-up in a paper machine in the pulp and paper industry traditionally requires a deposit control program including proper housekeeping to keep surfaces free of splashed stock, anti-microbial treatment of fresh water and additives, the use of biocides to reduce microbiological growth on the machine, and scheduled boil-outs to remove the deposits that do form.

WO 98/26807 discloses a method for cleaning and disinfecting a surface at least partly covered by a biofilm layer by contacting the biofilm with a cleaning composition comprising one or more hydrolases and contacting the biofilm with a bactericidal disinfecting composition. The composition comprising one or more hydrolases may comprise a *Bacillus* alpha-amylase, e.g. a *B stearothermophilus* alpha-amylase.

US 2004/018607 discloses several alpha-amylases and the use of alpha-amylases in hard surface cleaning. The document appears not to mention biofilm.

Bolton Declan et al. (1997) ENZYME and MICROBIAL TECHNOLOGY, vol. 20, no 5, page 340-343 discloses the purification and characterization of the alpha-amylase from *Bacillus flavothermus*. The document does not disclose any use of the alpha-amylase for cleaning purposes or for removal of biofilm.

Bacteria growing in biofilms are more resistant to antibiotics and disinfectants than planktonic cells and the resistance increases with the age of the biofilm. Bacterial biofilm also exhibits increased physical resistance towards desiccation, extreme temperatures or light. As mentioned, biofilm formation causes industrial, environmental and medical problems and the difficulties in cleaning and disinfection of bacterial biofilm with chemicals is a major concern in many industries. Furthermore, the trend towards milder disinfection and cleaning compositions to reduce their environmental impact may increase the insufficient cleaning of surfaces covered with biofilm.

It is an object of the present invention to provide improved methods for preventing or removing biofilm present on a surface.

### Brief Description of the Drawing

Fig. 1 shows a comparison of the percentage (%) of total hydrolysis of raw wheat starch for three different alpha-amylases.
Fig. 2 is a chromatogram comparing 1) Alpha-amylase A, 2) detergent alone 3) Alpha-amylase C biofilm removal.

### Summary of the Invention

The present invention relates to methods for preventing, removing, reducing, or disrupt biofilm formation on a surface, comprising contacting the surface with alpha-amylase derived from a bacterium.

The term "surface" is defined herein as any surface which may be covered by biofilm or is prone to biofilm formation. Examples of surfaces may be any hard surface such as metal, plastics, rubber, board, glass, wood, paper, concrete, rock, marble, gypsum and ceramic materials, which optionally are coated, for example, with paint or enamel; any soft surface such as fibers of any kind (e.g., yarns, textiles, vegetable fibers, rock wool, and hair); or any porous surfaces; skin (human or animal); keratinous materials (e.g., nails); and internal organs (*e.g.*, lungs). The hard surface can be present as a part of a cooling tower, water treatment plant, water tanks, dairy, food processing plant, chemical or pharmaceutical process plant, or medical device (e.g., catheters, orthopedic devices, implants). The porous surface can be present in a filter, e.g., a membrane filter.

The term "effective amount" is defined herein as the amount of one or more alpha-amylases that is sufficient to degrade a microbially-produced biofilm comprising alpha-1,4 glucosidic linkages. The effective amount of the one or more alpha-amylase will depend on factors including: the alpha-amylase(s) in question, whether the aim is preventing, removing, or reducing biofilms present on a surface, the period of time desirable for, e.g., degrading a microbially-produced biofilm. High amounts/concentrations of enzyme(s) will in general require shorter times of treatment, while low amounts/concentrations longer times. Further, for instance, preventing biofilm on a surface prone to biofilm formation will in general require lower amounts/concentrations of enzyme(s) than the actual removal of biofilm from a corresponding contaminated surface. However, typical effective usage levels are between 0.005 to 500 mg of alpha-amylase protein per L biofilm control solution, preferably between 0.01 - 100 mg of enzyme protein per L biofilm control solution. The term "biofilm control solution" refers to a solution used according to the invention for preventing, removing, reducing or disrupting biofilm present on a surface. The method of the invention may result in 10-10⁸-fold, preferably 10³-10⁶-fold biofilm reduction in terms of average plate count under the conditions indicated in Example 4 below.

### Detailed Description of the Invention

The present invention relates to improved methods of preventing, removing, or reducing biofilms present on a surface, comprising contacting the surface with an effective amount of an alpha-amylase as defined below. The methods of the present invention may be used to prevent, remove, reduce, or disrupt biofilm formation on a surface. One of ordinary skill in the art will recognize that such methods may be employed at different stages of biofim formation.

By using an alpha-amylase in an effective amount of surfaces improved biofilm prevention and/or removal is obtained, especially in those instances where some of the microbes present in the biofilm produce alpha-1,4 linked glucose polysaccharides such as amylose, amylopectin, mixtures of these two polysaccharides (i.e., starch), and glycogen.

In the first aspect the invention relates to a method for preventing or removing biofilm on a surface, comprising contacting the surface with an alpha-amylase derived from a bacterium. In a preferred embodiment the bacterial alpha-amylase is derived from a strain of *Bacillus.*

### Alpha-Amylase

The alpha-amylase used according to the invention is the AMY1048 alpha-amylase shown in SEQ ID NO: 4 herein, or an alpha-amylase having a degree of identity of at least 90%, such as at least 95%, at least 96%, at least 97%, at least 98% or at least 99% to any of the sequences shown in SEQ ID NO: 4 herein.

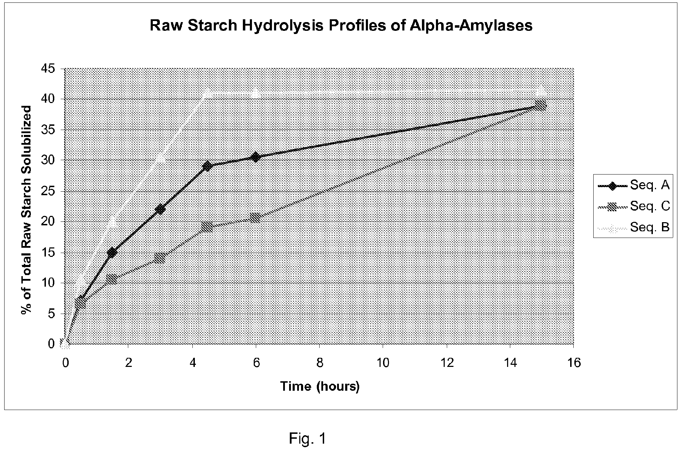

In a preferred embodiment the alpha-amylase has a percentage (%) of hydrolyzed raw starch that is higher than 15, preferably 25, especially 35, after 5 hours at 40°C, 3 mg enzyme protein per g starch, pH 8.0 (See Example 2 and Fig. 1).

In another preferred embodiment the alpha-amylase comprises Asn-Gly-Thr-Met-Met-Gln-Tyr-Phe-Glu-Trp in its N-terminal amino acid region. Examples of such alpha-amylase include Alpha-Amylase A and Alpha-Amylase B used in Example 2.

In a preferred embodiment, surfaces prone to biofilm formation may be subjected to the methods of the present invention as a preventative measure prior to any biofilm formation so no biofilm forms. Alternatively, at the first indication of biofim formation, the methods may be used to prevent further formation and to remove the biofim that has deposited on a surface. Furthermore, in situations where there is a heavy build-up of biofilm on a surface, the methods may be used to reduce the level of biofilm or to remove it partially or completely.

A biofilm may comprise an integrated community of one or two or more microorganisms or predominantly a specific microorganism (Palmer and White, 1997, Trends in Microbiology 5: 435-440; Costerton et al., 1987, Annual Reviews of Microbiology 41: 435-464; Mueller, 1994, TAPPI Proceedings, 1994 Biological Sciences Symposium 195-201). In the methods of the present invention, the one or more microorganisms may be any microorganism involved in biofilm formation including, but not limited to, aerobic bacteria or anaerobic bacteria (Gram positive and Gram negative), fungi (yeast or filamentous fungus), algae, and/or protozoa. Contemplated bacteria include bacteria selected from the group consisting of. *Pseudomonas* spp. including *Pseudomonas aeruginosa, Azotobacter vinelandii*, *Escherichia coli*, *Corynebacterium diphteriae*, *Clostridium botulinum, Streptococcus spp, Acetobacter, Leuconostoc, Betabacterium, Pneumococci, Mycobacterium tuberculosis, Aeromonas, Burkholderie, Flavobacterium, Salmonella*, *Staphylococcus.*

In a preferred embodiment, the microorganism is an aerobic bacterium. In a more preferred embodiment, the aerobic bacterium is an *Aeromonas* strain. In another more preferred embodiment, the aerobic bacterium is a *Burkholderie* strain. In another more preferred embodiment, the aerobic bacterium is a *Flavobacterium* strain. In another more preferred embodiment, the aerobic bacterium is a *Microbacterium* strain. In another more preferred embodiment, the aerobic bacterium is a *Pseudomonas* strain. In another more preferred embodiment, the aerobic bacterium is a *Salmonella* strain. In another more preferred embodiment, the aerobic bacterium is a *Staphylococcus* strain. In another more preferred embodiment, the aerobic bacterium is from the family *Enterobacteriaceae* (including *e.g., Escherichia coli*).

In a most preferred embodiment, the aerobic bacterium is *Burkholderie cepacia.* In another most preferred embodiment, the aerobic bacterium is a *Microbacterium imperiale* or *Mycobacterium tuberculosis*. In another most preferred embodiment, the aerobic bacterium is *Pseudomonas aeruginosa.* In another most preferred embodiment, the aerobic bacterium is *Pseudomonas fluorescens*. In another most preferred embodiment, the aerobic bacterium is *Pseudomonas oleovorans.* In another most preferred embodiment, the aerobic bacterium is *Pseudomonas pseudoalcaligenes*. In another most preferred embodiment, the aerobic bacterium is *Salmonella enteritidis.* In another most preferred embodiment, the aerobic bacterium is *Staphylococcus aureus.* In another most preferred embodiment, the aerobic bacterium is *Staphylococcus epidermidis.*

In another preferred embodiment, the microorganism is an anaerobic bacteria. In another more preferred embodiment, the anaerobic bacterium is a *Desulfovibrio* strain. In another most preferred embodiment, the anaerobic bacterium is *Desulfovibrio desulfuricans*.

In another preferred embodiment, the microorganism is a fungus such as a yeast or filamentous fungus. In another more preferred embodiment, the yeast is a *Candida* strain. In another most preferred embodiment, the yeast is *Candida albicans.*

As mentioned above the treatment time for preventing or removing biofilm will depend on the dosage of the alpha-amylase, and the level of biofilm on the surface or prone to the area in question, but should preferably be adapted to the time normally used for conventional treatment of biofilm with antibiotics, biocides, bactericides, fungicides, bleaching agents, surfactants, caustic, and/or biopolymer degrading agents. Consequently, the dosage of the alpha-amylase may be adjusted according to the time period used during conventional treatments. However, where the alpha-amylase treatment is a separate step in the processing, the dosage of the alpha-amylase used will depend on the time period desired to accomplish the treatment.

In terms of alpha-amylase activity, the appropriate dosage of alpha-amylase for preventing or removing biofilms will depend on the amount of biofilm on the surface or prone to the area in question. The skilled person may determine a suitable alpha-amylase unit dosage. The dosage may be expressed in alpha-amylase units. Alpha-amylase units may be determined as "KNU", using the assay described below in the "Materials & Methods"-section. Biofilm contaminated or prone areas are preferably treated for between 1 minute and 2 days, preferably between 10 minutes and 1 day, preferably between 1 hour and 15 hours, more preferably less that 10 hours, with an alpha-amylase dosage of between 0.005 to 500 mg of alpha-amylase protein per L biofilm control solution, preferably between 0.01 to 100 mg of alpha-amylase protein per L biofilm control solution.

The alpha-amylase may be part of a composition to be used in the methods of the present invention. The composition may be in any form suitable for the use in question, *e.g*., in the form of a dry powder, agglomerated powder, or granulate, in particular a non-dusting granulate, liquid, in particular a stabilized liquid, or protected alpha-amylase. Granulates and agglomerated powders may be prepared by conventional methods, e.g., by spraying the alpha-amylase onto a carrier in a fluid-bed granulator. The carrier may consist of particulate cores having a suitable particle size. The carrier may be soluble or insoluble, e.g., a salt (such as sodium chloride or sodium sulfate), sugar (such as sucrose or lactose), sugar alcohol (such as sorbitol), or starch. The alpha-amylase may be contained in slow-release formulations. Methods for preparing slow-release formulations are well known in the art. Liquid alpha-amylase preparations may, for instance, be stabilized by adding nutritionally acceptable stabilizers such as a sugar, sugar alcohol, or another polyol, and/or lactic acid or another organic acid according to established methods.

The composition may be augmented with one or more agents for preventing or removing the formation of the biofilm. These agents may include, but are not limited to, dispersants, surfactants, detergents, other enzymes, anti-microbials, and biocides.

In a preferred embodiment, the agent is a surfactant. The surfactant may be a non-ionic including semi-polar and/or anionic and/or cationic and/or zwitterionic surfactant.

Anionic surfactants contemplated include linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

Non-ionic surfactants contemplated include alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides").

The surfactants may be present at a level of from 0.1% to 60% by weight of the enzyme biofilm removal composition.

In a more preferred embodiment, the surfactant is sodium dodecyl sulfate, quaternary ammonium compounds, alkyl pyridinium iodides, Tween 80, Tween, 85, Triton X-100, Brij 56, biological surfactants, rhamnolipid, surfactin, visconsin, or sulfonates.

The formation of biofilm is generally accompanied by the production of exo-polymeric materials (polysaccharides, polyuronic acids, alginates, glycoproteins, and proteins) which together with the cells form thick layers of differentiated structures separated by water-filled spaces (McEldowney and Fletcher, 1986, Journal of General Microbiology 132: 513-523; Sutherland, Surface Carbohydrates of the Prokaryotic Cell, Academic Press, New York, 1977, pp. 27-96). In the methods of the present invention, the alpha-amylase composition may further comprise one or more other enzymes capable of degrading the exo-polymeric materials such as polysaccharides, polyuronic acids, alginates, glycoproteins, and proteins.

### Other enzyme activities

In a preferred embodiment, the one or more other enzymes may be selected from the group consisting of an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, oxididases, including carbohydrate oxidases, peroxidases, laccase, lipase, mannosidase, pectinolytic enzyme, peptidoglutaminase, phytase, polyphenoloxidase, proteolytic enzyme, ribonuclease, transglutaminase, or xylanase.

The other enzyme(s) may be selected according to the properties of the specific biofilm which is to be removed, or a combination of several enzymes having different enzyme activities may be used.

In a preferred embodiment, the other enzyme is selected from the group consisting of 1,2-1,3-alpha-D-mannan mannohydrolase, 1,3-beta-D-xylan xylanohydrolase, 1,3-beta-D-glucan glucanohydrolase, 1,3(1,3;1,4)-alpha-D-glucan 3-glucanohydrolase, 1,3(1,3;1,4)-beta-D-glucan 3(4)-glucanohydrolase, 1,3-1,4-alpha-D-glucan 4-glucanohydrolase, 1,4-alpha-D-glucan glucanehydrolase, 1,4-alpha-D-glucan glucohydrolase, 1,4-(1,3:1,4)-beta-D-glucan 4-glucanohydrolase, 1,4-beta-D-glucan glucohydrolase, 1,4-beta-D-xylan xylanohydrolase, 1,4-beta-D-mannan mannanohydrolase, 1,5-alpha-L-arabinan 1,5-alpha-L-arabinanohydrolase, 1,4-alpha-D-glucan maltohydrolase, 1,6-alpha-D-glucan 6-glucanohydrolase, 2,6-beta-D-fructan fructanohydrolase, alpha-Dextrin 6-glucanohydrolase, alpha-D-galactoside galactohydrolase, alpha-D-glucoside glucohydrolase, alpha-D-mannoside mannohydrolase, acylneuraminyl hydrolase, *Aerobacter*-capsular-polysaccharide galactohydrolase, beta-D-fructofuranoside fructohydrolase, beta-D-fucoside fucohydrolase, beta-D-fructan fructohydrolase, beta-D-galactoside galactohydrolase, beta-D-glucoside glucohydrolase, beta-D-glucuronoside, glucuronosohydrolase, beta-D-mannoside mannohydrolase, beta-N-acetyl-D-hexosaminide N-acetylhexosamino hydrolase, cellulose-sulfate sulfohydrolase, collagenase, dextrin 6-alpha-D-glucanohydrolase, glycoprotein-phosphatidylinositol phosphatidohydrolase, hyaluronate 4-glycanohydrolase, hyaluronoglucuronidase, pectin pectylhydrolase, peptidoglycan N-acetylmuramoylhydrolase, phosphatidylcholine 2-acylhydrolase, phosphatidylcholine 1-acylhydrolase, poly(1,4-alpha-D-galacturonide), poly(1,4-(N-acetyl-beta-D-glucosaminide))-glycanohydrolase, sucrose alpha-glucosidase, triacylglycerol acylhydrolase, and triacylglycerol protein-acylhydrolase.

### Proteolytic enzyme

The other enzyme may be any enzyme having proteolytic activity under the actual process conditions. Thus, the enzyme may be a proteolytic enzyme of plant origin, e.g., papain, bromelain, ficin, or of animal origin, e.g., trypsin and chymotrypsin, or of microbial origin, i.e., bacterial, yeast, or filamentous fungal. It is understood that any mixture of various proteolytic enzyme may be applicable in the process of the invention.

In another preferred embodiment, the other enzyme is a proteolytic enzyme such as a serine protease, a metalloprotease, or an aspartate protease.

A sub-group of the serine proteases are commonly designated as subtilisins. A subtilisin is a serine protease produced by Gram-positive bacteria or fungi. The amino acid sequence of a number of subtilisins have been determined, including at least six subtilisins from *Bacillus* strains, namely, subtilisin 168, subtilisin BPN, subtilisin Carlsberg, subtilisin DY, subtilisin amylosacchariticus, and mesentericopeptidase, one subtilisin from an actinomycetales, thermitase from *Thermoactinomyces vulgaris,* and one fungal subtilisin, proteinase K from *Tritirachium album.* A further subgroup of the subtilisins, subtilases, has been recognised more recently. Subtilases are described as highly alkaline subtilisins and comprise enzymes such as subtilisin PB92 (MAXACAL^{®}, Gist-Brocades NV), subtilisin 309 (SAVINASE^{®}, Novozymes A/S), and subtilisin 147 (ESPERASE^{®}, Novozymes A/S).

A "subtilisin variant or mutated subtilisin protease" is defined herein as a subtilisin that has been produced by an organism which is expressing a mutant gene derived from a parent microorganism which possessed an original or parent gene and which produced a corresponding parent enzyme, the parent gene having been mutated in order to produce the mutant gene from which said mutated subtilisin protease is produced when expressed in a suitable host. These mentioned subtilisins and variants thereof constitute a preferred class of proteases which are useful in the method of the invention. An example of a useful subtilisin variant is a variant of subtilisin 309 (SAVINASE^{®}) wherein, in position 195, glycine is substituted by phenylalanine (G195F or ¹⁹⁵Gly to ¹⁹⁵Phe).

Commercially available proteases may be used in the methods of the present invention. Examples of such commercial proteases are ALCALASE^{®} (produced by submerged fermentation of a strain of *Bacillus licheniformis*), ESPERASE^{®} (produced by submerged fermentation of an alkalophilic species of *Bacillus*), RENNILASE^{®} (produced by submerged fermentation of a non-pathogenic strain of *Mucor miehei*), SAVINASE^{®} (produced by submerged fermentation of a genetically modified strain of *Bacillus*), e.g., the variants disclosed in the International Patent Application published as WO 92/19729, and DURAZYM^{®} (a protein-engineered variant of SAVINASE^{®}), POLARZYME™, EVERLASE™. All the above-mentioned commercial proteases are available from Novozymes A/S, DK-2880 Bagsvaerd, Denmark.

Other preferred serine proteases are proteases from *Aspergillus, Bacillus* such as *Bacillus alcalophilus, Bacillus cereus, Bacillus vulgatus, Bacillus mycoide, Rhizopus,* and subtilins from *Bacillus,* especially proteases from the species *Nocardiopsis* such as *Nocardiopsis natto Nocardiopsis dassonvillei* (see, WO 88/03947), especially proteases from the species *Nocardiopsis sp.* NRRL 18262, and *Nocardiopsis dassonvillei* NRRL 18133. Yet other preferred proteases are the serine proteases from mutants of *Bacillus* subtilisins disclosed in the International Patent Application No. PCT/DK89/00002 and WO 91/00345, and the proteases disclosed in EP 415 296.

Another preferred class of proteases is the metalloproteases of microbial origin. Conventional fermented commercial metalloproteases may be used in the methods of the present invention such as is NEUTRASE^{®} (Zn) (produced by submerged fermentation of a strain of *Bacillus subtilis*), available from Novozymes A/S, DK-2880 Bagsvaerd, Denmark; BACTOSOL^{®} WO and BACTOSOL^{®} SI, available from Sandoz AG, Basle, Switzerland; TOYOZYME^{®}, available from Toyo Boseki Co. Ltd., Japan; and PROTEINASE K^{®} (produced by submerged fermentation of a strain of *Bacillus sp.* KSM-K16), available from Kao Corporation Ltd., Japan.

The protease may be used in a dosage of between 0.005 to 500 mg enzyme protein per L biofilm control solution, preferably between 0.01 to 100 mg enzyme protein per L biofilm control solution.

### Lipases

In another preferred embodiment, the other enzyme is a lipase, especially a microbial lipase. As such, the lipase may be selected from yeast, e.g., *Candida*; bacteria, e.g., *Pseudomonas* or *Bacillus;* or filamentous fungi, e.g., *Humicola* or *Rhizomucor.* More specifically, suitable lipases may be the *Rhizomucor miehei* lipase (*e.g.,* prepared as described in EP 238 023), *Thermomyces lanuginosa* lipase e.g., prepared as described in EP 305 216, *Humicola insolens* lipase, *Pseudomonas stutzeri* lipase, *Pseudomonas cepacia* lipase, *Candida antarctica* lipase A or B, or lipases from *rGPL, Absidia blakesleena, Absidia corymbifera, Fusarium solani, Fusarium oxysporum, Penicillum cyclopium, Penicillum crustosum, Penicillum expansum, Rhodotorula glutinis, Thiarosporella phaseolina, Rhizopus microsporus, Sporobolomyces shibatanus, Aureobasidium pullulans, Hansenula anomala, Geotricum penicillatum, Lactobacillus curvatus, Brochothrix thermosohata, Coprinus cinerius, Trichoderma harzanium, Trichoderma reesei, Rhizopus japonicus,* or *Pseudomonas plantari.* Other examples of suitable lipases may be variants of any one of the lipases mentioned above, e.g., as described in WO 92/05249 or WO 93/11254.

Examples of commercially available lipases include: LIPOLASE™, LIPOLASE ULTRA™, LIPOPRIME™, LIPEX™ from Novozymes, Denmark).

The lipase may be used in a dosage of between 0.005 to 500 mg enzyme protein per L biofilm control solution, preferably between 0.01 to 100 mg enzyme protein per L biofilm control solution.

### Cellulases

In another preferred embodiment, the other enzyme is a cellulase or cellulolytic enzyme, which refers to an enzyme which catalyses the degradation of cellulose to glucose, cellobiose, triose and other cellooligosaccharides. Preferably, the cellulase is an endoglucanase, more preferably a microbial endoglucanase, especially a bacterial or fungal endoglucanase. Examples of bacterial endoglucanases are endoglucanases obtained from or producible by bacteria from the group of genera consisting of *Pseudomonas* or *Bacillus lautus*.

The cellulase or endoglucanase may be an acid, neutral, or alkaline cellulase or endoglucanase, i.e., exhibiting maximum cellulolytic activity in the acid, neutral or alkaline pH range, respectively. Accordingly, a useful cellulase or endoglucanase is an acid cellulase or endoglucanase, preferably a fungal acid cellulase or endoglucanase, more preferably a fungal acid cellulase or endoglucanse enzyme with substantial cellulolytic activity under acidic conditions, which is obtained from or producible by fungi from the group consisting of *Trichoderma, Actinomyces, Myrothecium, Aspergillus,* and *Botrytis.*

A preferred acid cellulase or endoglucanase is obtained from the group consisting of *Aspergillus niger, Aspergillus oryzae, Botrytis cinerea, Myrothecium verrucaria, Trichoderma longibrachiatum, Trichoderma reesei,* and *Trichoderma viride*.

Another useful cellulase or endoglucanase is a neutral or alkaline cellulase or endoglucanse, preferably a fungal neutral or alkaline cellulase or endoglucanse, more preferably a fungal alkaline cellulase or endoglucanase with substantial cellulolytic activity under alkaline conditions, which is obtained from fungi selected from the group consisting of *Acremonium, Aspergillus, Chaetomium, Cephalosporium, Fusarium, Gliocladium, Humicola, Irpex, Myceliophthora, Mycogone, Myrothecium, Papulospora, Penicillium, Scopulariopsis, Stachybotrys,* and *Verticillium.*

A preferred alkaline cellulase or endoglucanase is obtained from the group consisting of *Cephalosporium sp., Fusarium oxysporum, Humicola insolens,* or *Myceliopthora thermophila,* or preferably from the group consisting of *Cephalosporium* sp., RYM-202, *Fusarium oxysporum,* DSM 2672, *Humicola insolens,* DSM 1800, or *Myceliopthora thermophila,* CBS 117.65.

In another preferred embodiment, the other enzyme is a xylanase such as an endo-1,3-beta-xylosidase (EC 3.2.1.32), xylan 1,4-beta-xylosidase (EC 3.2.1.37), and alpha-L-arabinofuranosidase (EC 3.2.1.55). Preferably the xylanase is obtained from *Aspergillus aculeatus* (an enzyme exhibiting xylanase activity, which enzyme is immunologically reactive with an antibody raised against a purified xylanase derived from *Aspergillus aculeatus* CBS 101.43, see, for example, WO 94/21785); *Aspergillus oryzae* (see, for example, SU 4610007); *Aureobasidium pullulans* (see, for example, EP 0 373 107 A2); *Bacillus circulans* (WO 91/18978); *Bacillus pumilus* (see, for example, WO 92/03540); *Bacillus stearothermophilus* (see, for example, WO 91/18976, WO 91/10724); *Bacillus sp. AC13* (especially the strain NCIMB 40482, see, for example, WO 94/01532); *Humicola insolens* (see, for example, WO 92/17573); *Rhodothermus* (see, for example, WO 93/08275); *Streptomyces lividans* (see, for example, WO 93/03155); *Streptomyces viridosporus* (see, for example, EP 496 671 A*)*; *Bacillus licheniformis* (see, for example, JP 9213868); *Thermoascus aurantiacus* (see, for example, US patent 4,966,850); *Trichoderma longibrachiatum* and *Chainia sp.* (see, for example, EP 0 353 342 A1); *Trichoderma harzianum* and *Trichoderma reseei* (see, for example, US patent 4,725,544); *Thermomyces lanuginosus* (see, for example, EP 0 456 033 A2); *Thermomonospora fusca* (see, for example, EP 0 473 545 A2); *Trichoderma longibrachiatum* (see W.J.J. van den Tweel et al., Eds., Stability of Enzymes, Proceedings of an International Symposium held in Maastrich, The Netherlands, 22-25 November 1992, Fisk, R.S. and Simpson, pp.323-328); *Dictyoglomus* (see, for example, WO 92/18612); *Streptomyces* (see, for example, US patent 5,116,746); and/or *Thermotoga* (see, for example, WO 93/19171). Other examples of suitable xylanases may be variants (derivatives or homologues) of any one of the above-noted enzymes having xylanolytic activity.

Examples of commercially available cellulase containing products include: NOVOZYM™ 342, CELLUZYME™, CAREZYME™, RENOZYME™ (all Novozymes, Denmark).

The cellulase may be used in a dosage of between 0.005 to 500 mg enzyme protein per L biofilm control solution, preferably between 0.01 to 100 mg enzyme protein per L biofilm control solution.

### Pectinases

In another preferred embodiment, the other enzyme is a pectinase such as a polygalacturonase (EC 3.2.1.15), pectinesterase (EC 3.2.1.11), or pectin lyase (EC4.2.2.10). A suitable source organism for pectinases may be *Aspergillus niger.*

In another preferred embodiment, the other enzyme in the alpha-amylase composition comprises a hydrolytic enzyme composition produced by a strain of the fungus *Aspergillus aculeatus*, preferably *Aspergillus aculeatus*, CBS 101.43. It is known that this strain produces an enzyme composition comprising pectinolytic and a range of hemicellulolytic enzyme activities.

Examples of commercially available cellulase containing products include: BioPrep™, SCOURZYME™ and PECTAWASH™ (Novozymes, Denmark).

The pectinase may be used in a dosage of between 0.005 to 500 mg enzyme protein per L biofilm control solution, preferably between 0.01 to 100 mg enzyme protein per L biofilm control solution.

### Oxidoreductase

In another embodiment of the invention the alpha-amylase is combined with an oxidoreductase, such as an oxidase, peroxidase, or laccase.
a) Laccases act on molecular oxygen and yield water (H₂O) without any need for peroxide (e.g. H₂O₂),
b) Oxidases act on molecular oxygen (O₂) and yield peroxide (H₂O₂), and
c) Peroxidases act on peroxide (e.g. H₂O₂) and yield water (H₂O).

Examples of laccases (E.C. 1.10.3.2) include laccases derived from a strain of *Polyporus* sp., in particular a strain of *Polyporus pinsitus* or *Polyporus versicolor,* or a strain of *Myceliophthora* sp., in particular M. *thermophila,* a strain of *Scytalidium sp.,* in particular S. *thermophilium,* a strain of *Rhizoctonia* sp., in particular *Rhizoctonia praticola* or *Rhizoctonia solani,* or a strain of a *Rhus* sp., in particular *Rhus vernicifera.* The laccase may also be derived from a fungus such as *Collybia, Fomes, Lentinus, Pleurotus, Aspergillus, Neurospora, Podospora, Phlebia,* e.g. *P. radiata* (WO 92/01046), *Coriolus* sp., e.g., C. *hirsitus* (JP 2-238885), or *Botrytis*.

In specifically contemplated embodiments, the laccase may be selected from the group consisting of: the *Polyporus pinisitus* laccase (also called *Trametes villosa* laccase) described in WO 96/00290, the *Myceliophthora thermophila* laccase described in WO 95/33836, the *Scytalidium thermophilium* laccase described in WO 95/33837, the *Pyricularia oryzae* laccase which can be purchased from SIGMA under the trade name SIGMA no. L5510, the *Coprinus cinereus* laccase described in WO 96/06930, and the *Rhizoctonia solani* laccase described WO 95/07988.

Examples of peroxidases (1.11.1.7) include peroxidases derived from plants (e.g., horseradish peroxidase) or micro-organisms including fungi and bacteria, such as a strain of *Coprinus* sp., such as *Coprinus cinereus* or *Coprinus macrorhizus,* or bacteria such as *Bacillus,* such as *Bacillus pumilus.*

In specifically contemplated embodiments, the peroxidase may be selected from the group consisting of: the *Coprinus cinereus* IFO8371 peroxidase or variants thereof described in WO 95/10602, and the haloperoxidase originating from a strain of *Curvularia verruculosa* CBS 147.63 described in WO 97/04102.

Contemplated oxidases include especially carbohydrate oxidases, which are enzymes classified under EC 1.1.3. Carbohydrate oxidases include glucose oxidase (E.C. 1.1.3.4), hexose oxidase (E.C. 1.1.3.5) xylitol oxidase, galactose oxidase (E.C. 1.1.3.9), pyranose oxidase (E.C. 1.1.3.10), alcohol oxidase (E.C. 1.1.3.13).

Carbohydrate oxidases may be derived from any origin, including, bacterial, fungal, yeast or mammalian origin.

Examples of glucose oxidases include glucose oxidases derived from *Aspergillus* sp., such as a strain of *Aspergillus niger,* or from a strain of *Cladosporium* sp. in particular *Cladosporium oxysporum,* especially *Cl. oxysporum* CBS 163 described in WO 95/29996.

Examples of hexose oxidases include hexose oxidases produced by the red sea-weed *Chondrus crispus* (commonly known as Irish moss) (Sullivan and Ikawa, (1973), Biochim. Biophys. Acts, 309, p. 11-22; Ikawa, (1982), Meth. in Enzymol. 89, carbohydrate metabolism part D, 145-149) that oxidizes a broad spectrum of carbohydrates and the red sea-weed *Iridophycus flaccidum* that produces easily extractable hexose oxidases, which oxidize several different mono- and disaccharides (Bean and Hassid, (1956), J. Biol. Chem, 218, p. 425; Rand et al. (1972, J. of Food Science 37, p. 698-710).

The oxidoreductase may be used in a dosage of between 0.005 to 500 mg enzyme protein per L biofilm control solution, preferably between 0.01 to 100 mg enzyme protein per L biofilm control solution.

In a final aspect the invention relates to the use of an alpha-amylase having the sequence shown in SEQ ID NO:4, or an alpha amylase having at least 90 % identity to SEQ ID NO 4 for preventing, removing, reducing, or disrupting biofilm formation on a surface. In a preferred embodiment the alpha-amylase is one mentioned above in the "Alpha-Amylase" section.

The present invention is further described by the following examples which should not be construed as limiting the scope of the invention.

### Materials & Methods

Chemicals used as buffers and reagents were commercial products of at least reagent grade.

### Enzymes:

Alpha-amylase A is a variant alpha-amylase of the parent *Bacillus* sp. alpha-amylase disclosed as SEQ ID NO: 2 in WO 00/60060. The amino acid sequence of said alpha-amylase has the following six amino acid deletions/substitutions:
D183*+G184*+R118K+N195F+R320K+R458K.

The variant is also disclosed in WO 01/66712. The alkaline alpha-amylase was produced in batch 03AGE014-4.

Alpha-amylase B is derived from a strain of *Bacillus flavothermus* and is disclosed in SEQ ID NO: 4.

Alpha-amylase C is derived from a strain of *Bacillus licheniformis* and is shown as SEQ ID NO: 6 in WO 99/19467.

Protease E is a *Bacillus clausii* (old name: *Bacillus lentus* C360 = NCIB 10309) subtilisin having a M222S substitution covered by EP patent no. 396,608-B1 (Available on request from Novozymes, Denmark).

Lipase A is a lipase variant derived from *Humicola lanuginosa* strain DSM 4109 having the following mutations: T231R, N233R disclosed in US patent no. 6,939,702-B (Available on request from Novozymes).

Cellulase A is a multi-component cellulase from *Humicola insolens* (Available on request from Novozymes, Denmark).

### Bacterial strains.

*Bacillus subtilis* obtained from ATCC 10774.
E. coli ATCC #11229 and ATCC #25922

**Biofilm medium.** Tryptic Soy Broth (TSB, purchased from VWR, P/N DF0370-07) medium was prepared according to the manufacturer's instructions, then diluted to 5% with water. 2 ml of trace elements were added per liter.

**Agar.** Tryptic Soy Agar (TSA, purchased from VWR, P/N DF0369-17) was used as per the manufacturer's directions.

**Trace element solution.** Per liter: 1.5 g CaCl₂, 1.0 g FeSO₄7.H₂O, 0.35 g MnSO₄.2H₂O, 0.5 g, NaMoO₄.

**Stainless steel coupons.** Stainless steel coupons No. 304 were obtained from Metal Samples Company (Munford, AL).

**BioLC Ion Chromatography System.** The IC system consisted of the following components:
GP50 Gradient Pump (P/N 059493)
ED50A Electrochemical Detector (P/N 059499)
AS50 Temperature Controlled Autosampler (P/N 056565)
Electrochemical Cell for Integrated Amperometry, complete with Gold Electrode and Ag/AgCl Reference Electrode (P/N 060386)
Chromelion Data Control Software CHM-1-IC (P/N 060930)

**CDC Biofilm Reactor.** Purchased from Biosurface Technologies, Inc. (P/N CBR 90-2) complete with polycarbonate coupons (24 for each reactor, P/N RD 128-PC).

**Detergent Cleaner Base.** Obtained from Weiman Products (IL, USA) as Burnishine ME **-** multiple enzyme detergent. The enzymes were denatured prior to use by heating in a microwave on high setting for 1 minute.

### Methods:

### Alpha-amylase activity (KNU)

The amylolytic activity may be determined using potato starch as substrate. This method is based on the break-down of modified potato starch by the enzyme, and the reaction is followed by mixing samples of the starch/enzyme solution with an iodine solution. Initially, a blackish-blue color is formed, but during the break-down of the starch the blue color gets weaker and gradually turns into a reddish-brown, which is compared to a colored glass standard.

One Kilo Novo alpha amylase Unit (KNU) is defined as the amount of enzyme which, under standard conditions (i.e. at 37°C +/- 0.05; 0.0003 M Ca²⁺; and pH 5.6) dextrinizes 5260 mg starch dry substance Merck Amylum solubile.

A folder EB-SM-0009.02/01 describing this analytical method in more detail is available upon request to Novozymes A/S, Denmark, which folder is hereby included by reference.

### Determination of degree of identity between two sequences

For purposes of the present invention, the degree of identity between two amino acid sequences is determined by the Clustal method (Higgins, 1989, CABIOS 5: 151-153) using the LASERGENE™ MEGALIGN™ software (DNASTAR, Inc., Madison, WI) with an identity table and the following multiple alignment parameters: Gap penalty of 10, and gap length penalty of 10. Pairwise alignment parameters were Ktuple=1, gap penalty=3, windows=5, and diago-nals=5.

### EXAMPLES

### Example 1

### Biofilm removal using Alpha-amylase A and Alpha-Amylase C

Biofilm reactors consisted of a 400 ml beaker, a magnetic stirrer, and 2 stainless steel coupons. The coupons are taped vertically to the sides of the beaker so that the bottom edge of the coupon rested on the bottom of the beaker. A stir bar is added and the beakers are covered with a circle of aluminum foil and autoclaved. 200 ml of sterile biofilm medium is added to each beaker. To prepare the inoculum, each bacterial strain (from *Bacillus subtilis)* is grown overnight at 28°C on plate count agar. Using a sterile swab, each is suspended in sterile water to an OD₆₈₆ of 0.100 and then diluted additionally to 10⁻¹. Each assay consisted of 4 control beakers without enzyme, 2 beakers with 50 mg of enzyme protein per liter of solution, and 2 beakers with 100 mg of enzyme protein per liter of solution. Beakers are first incubated at 37°C overnight with stirring to grow the biofilm on the stainless steel coupons. Following this incubation step, the enzymes are added in the 2 dosages noted above as per the table below and incubated for an additional 2 hours at 40°C. Thereafter, each beaker and stainless steel coupon is rinsed carefully with sterile water, stained with crystal violet, rinsed with steile water, the remaining biofilm solubilized with acetic acid, and the absorbance of an aliquot of each solution is measured at 600nm using a spectrophotometer. The measured absorbances of the solutions provide a direct indication of the amount of biofilm remaining on the stainless steel coupons. A low absorbance corresponds to a good enzyme effect and little remaining biofilm, whereas a high absorbance corresponds to a poor (or lack thereof) enzyme effect and considerable remaining biofilm.

| **Sample #** | **Enzyme Used** | **Enzyme Protein Conc.** |
|---|---|---|
| 1 | Alpha-amylase A | 50 mg per L & 100 mg per L |
| 2 | Alpha-Amylase C | 50 mg per L & 100 mg per L |
| 3 | No enzyme (control) | NA |

### Example 2

### Raw starch solubilization using Alpha-Amylase A, B, and C

The rate at which various alpha-amylases solubilize raw, unhydrated wheat starch was measured. Alpha-Amylase A, B and C, respectively, were used in the study.

Twenty five milliliters of a 1% raw wheat starch solution with pH 8 tris buffer and 15°dH was poured into a tube with lid and placed in a 40°C water bath. The starting level of "reducing ends" was measured prior to addition of enzyme. The enzyme concentration used in the study was 3 mg enzyme protein per g raw wheat starch. One milliliter samples were taken out at different times. Twenty microliters of 1 M HCl was added prior to incubation at 99°C for 10 minutes. The combination of acid and heat inactivates the amylase. Then 20 microL 1 M NaOH was added to make sure the sample was no longer acidic. The sample was then diluted, incubated with color reagent (PHABH, potassium sodium tartrate, NaOH) at 95°C for 10 minutes and finally centrifuged before measuring the OD at 410 nm on the supernatant. The control (100% hydrolyzed starch) was made by incubating a solution of 1% raw wheat starch in 1 M HCl in an oven at 110°C for 4 hours. This treatment was used to calculate the maximum amount of glucose which could be produced per gram of the raw wheat starch. This value was set to 100% in the graph shown in Fig. 1.

For Alpha-Amylase A and B it can be seen that the initial rate of raw wheat starch solubilization observed within the first 5 hours is significantly more rapid than for Alpha-Amylase C. This resulted in a greater percentage of the starch being solubilized by the former two alpha-amylases vs. for the latter over this time period.

### Example 3

### Biofilm removal using Alpha-amylase A and C in combination with protease E and detergent

A mono-component biofilm of *Escherichia coli* (ATCC #11229) is grown on polycarbonate coupons in a pre-sterilized CDC biofilm reactor. At the start of the experiment, cultures of *E. coli* were grown on tryptic soy agar (TSA) overnight at 37°C. The next morning, a single colony was picked from the plate using a 1 microL sterile inoculation loop and added to a solution of 40 g TSB/liter of water. This solution was incubated at 37°C overnight to grow up the culture. The following day, 1 milliliter of this culture was added to 400 milliliters of minimal media (0.30 g TSB/liter sterile water) contained in the CDC biofilm reactor. The solution was slowly stirred at 130 rpm and grown for 2 days at 22°C in a non fed batch mode. After the 2 day growth period, the coupon holder rods and coupons were removed from the reactor, rinsed in sterile dilution water to remove planktonic cells, the coupons were carefully removed from the rods and then incubated at 40°C for 1 hour in the following solutions (30 milliliters each).
A: Detergent cleaner base alone, 0.21 g detergent in sterile water
B: Detergent cleaner base, 0.21 g + 0.51 mg enzyme protein Protease E + 0.06 mg enzyme protein Alpha-amylase A
C: Detergent cleaner base, 0.21 g + 0.51 mg enzyme protein Protease E + 0.16 mg enzyme protein Alpha-amylase C.

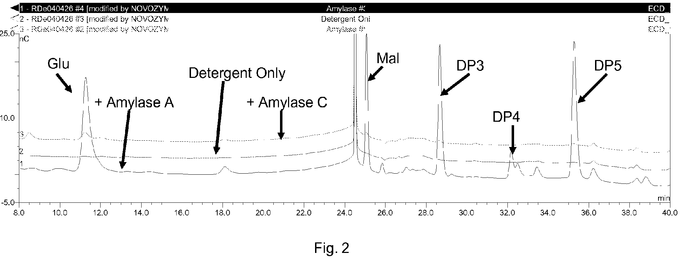

Following the incubation step, the coupons were removed. The solutions were filtered through 0.45□micro m Nylon syringe filters and their sugar contents measured by Ion Chromatography. PA100 guard and analytical columns (P/N 043055) were used for the separation. A mobile phase gradient between 60/40 deionized water/100mM NaOH and 100% 100mM NaOH/1M Sodium Acetate (exponential gradient started 10 minutes into the separation and ended at 85 minutes) was used to effect the separation. Fig. 2 shows an overlay of the 3 chromatograms generated in this experiment. When Alpha-Amylase A is used, a significantly higher level of low molecular weight sugars (glucose = glu, maltose = mal, maltotriose = DP3, maltotetraose = DP4, maltopentaose = DP5) were generated versus with the detergent alone or with Alpha-Amylase C. This indicated an increased level of biofilm removal through increased breakdown of the amylopectin exopolysaccharides produced by the *Ecoli* bacteria.

### Example 4

### Biofilm removal using Alpha-amylase A and C in combination with protease E, Cellulase A, Lipase A and detergent.

Two CDC biofilm reactors fitted with polycarbonate coupons were autoclaved and filled with sterile 1/10 strength tryptic soy broth (TSB, 3g/liter strength) and inoculated with 1 ml log phase culture of *Escherichia coli* (ATCC # 25922). The initial cell count in the reactors averaged to 5x10⁸ cfu/mL. Both reactors were operated for 24 hours in batch mode at 37°C (no inflow or outflow). After this period, continuous flow of the 1/10 TSB was started at a flowrate of 12 ml/min at 37°C. The *E. coli* biofilm was grown for 4 days. After this time, 1 rod from each reactor (labeled as reactor 1 or 2) was pulled and placed into sterile glass beakers containing two hundred milliliters of the following filter-sterilized solutions.
A: Detergent cleaner base alone, 1.4 g detergent in sterile water
B: Detergent cleaner base, 1.4 g + 3.4 mg enzyme protein Protease E + 0.48 mg enzyme protein Lipase A + 0.23 mg enzyme protein Cellulase A + 0.40 mg enzyme protein Alpha-amylase A
C: Detergent cleaner base, 1.4 g + 3.4 mg enzyme protein Protease E+ 0.48 mg enzyme protein Lipase A + 0.23 mg enzyme protein Cellulase A + 0.40 mg enzyme protein Alpha-amylase C.

The solutions in each of the beakers were incubated at 40°C with moderate stirring for 30 minutes, after which each of the rods were gently rinsed in sterile water. Finally, *E. coli* was enumerated on 2 of the 3 coupons from each rod using tryptic soy agar (TSA). The average plate count results obtained from the study were as follows:

| Treatment | Average log₁₀ cfu/cm² on coupons |
|---|---|
| A | 4x10⁷ |
| B | 3x10⁴ |
| C | 2x10⁵ |

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

## Claims

1. A method for preventing, removing, reducing or disrupting biofilm present on a surface, comprising contacting the surface with an alpha-amylase having the sequence shown in SEQ ID NO:4, or an alpha amylase having at least 90 % identity to SEQ ID NO 4.

2. The method of claim 1, wherein the alpha-amylase is derived from a strain of *Bacillus.*

3. The method of claims 1 or 2, wherein the biofilm contaminated or prone surface is contacted for between 1 minute and 2 days.

4. The method of any of claims 1-3, wherein further a surfactant is present.

5. The method of any of claims 1-4, wherein the alpha-amylase comprises Asn-Gly-Thr-Met-Met-Gln-Tyr-Phe-Glu-Trp in its N-terminal amino acid region.

6. The method of any of claims 1-5, wherein the alpha-amylase is used in a concentration of between 0.005-500 mg enzyme protein, preferably between 0.01-100 mg of enzyme protein per L biofilm control solution.

7. The method of any of claims 1-6, wherein the alpha-amylase has a percentage (%) of hydrolyzed starch that is higher than 15, preferably 25, especially 35, after 5 hours at 40°C, 3 mg enzyme protein per g starch, pH 8.0.

8. The method of any of claims 1-7, wherein further enzymes are present, which enzymes are selected from the group consisting of an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, lipase, mannosidase, oxidoreductases, pectinolytic enzyme, peptidoglutaminase, peroxidase, phytase, polyphenoloxidase, proteolytic enzyme, ribonuclease, transglutaminase, or xylanase.

9. The method of claim 8, wherein the pectinolytic enzyme is selected from the group consisting of pectintranseliminase, polygalactutonase, pectineesterase.

10. The method of claim 9, wherein the cellulase is a multi-component cellulase preparation or an endoglucanase, preferably a *Humicola* endoglucanase, especially a *Humicola insolens* endoglucanase, or an endoglucanase, preferably a *Thielavia* endoglucanase, preferably a *Thielavia terrestris* endoglucanase, or a variant thereof.

11. The method of claim 10, wherein the proteolytic enzyme is a protease, preferably a serine-protease, especially derived from a strain of *Bacillus,* such as *Bacillus lentus* or *Bacillus clausii,* or a variant thereof.

12. The method of any of claims 1-11, wherein further one or more agents selected from the group consisting of dispersants, surfactants, anti-microbials, and biocides, are present.

13. The method of any of claims 1-12, wherein the surface is a hard, soft, or porous surface.

14. The method of claim 13, wherein the surface is a membrane.

15. The method of any of claims 1-14, wherein the biofilm removal is done at a temperature between 10-70°C, preferably 40-60°C.

16. Use of an alpha-amylase as defined in any of claims 1-15 for prevention or removal of biofilm from surfaces.

17. The use of claim 16, wherein further enzymes and/or agents as defined in claims 1 - 15 are used.

## Patentansprüche

1. Verfahren zum Verhindern, Entfernen, Verringern oder Unterbrechen von auf einer Oberfläche vorhandenem Biofilm, umfassend Inkontaktbringen der Oberfläche mit einer alpha-Amylase mit der in SEQ ID NO:4 gezeigten Sequenz, oder einer alpha-Amylase mit mindestens 90% Identität zu SEQ ID NO: 4.

2. Verfahren nach Anspruch 1, wobei die alpha-Amylase aus einem Stamm von *Bacillus* abgeleitet ist.

3. Verfahren nach Ansprüchen 1 oder 2, wobei die mit Biofilm kontaminierte oder anfällige Oberfläche für zwischen 1 Minute und 2 Tagen kontaktiert wird.

4. Verfahren nach einem beliebigen der Ansprüche 1-3, wobei weiterhin ein oberflächenaktives Mittel vorhanden ist.

5. Verfahren nach einem beliebigen der Ansprüche 1-4, wobei die alpha-Amylase Asn-Gly-Thr-Met-Met-Gln-Tyr-Phe-Glu-Trp in ihrer N-terminalen Aminosäureregion umfasst.

6. Verfahren nach einem beliebigen der Ansprüche 1-5, wobei die alpha-Amylase in einer Konzentration von zwischen 0,005-500 mg Enzymprotein, bevorzugt zwischen 0,01-100 mg Enzymprotein pro L Biofilmkontrolllösung verwendet wird.

7. Verfahren nach einem beliebigen der Ansprüche 1-6, wobei die alpha-Amylase eine Prozentzahl (%) an hydrolysierter Stärke aufweist, die höher als 15, bevorzugt 25, besonders 35, nach 5 Stunden bei 40°C, 3 mg Enzymprotein pro g Stärke, pH 8,0 ist.

8. Verfahren nach einem beliebigen der Ansprüche 1-7, wobei weitere Enzyme vorhanden sind, welche Enzyme ausgewählt sind aus der Gruppe bestehend aus einer Aminopeptidase, Amylase, Carbohydrase, Carboxypeptidase, Katalase, Cellulase, Chitinase, Cutinase, Cyclodextringlycosyltransferase, Desoxyribonuklease, Esterase, alpha-Galactosidase, beta-Galactosidase, Glucoamylase, alpha-Glucosidase, beta-Glucosidase, Haloperoxidase, Invertase, Laccase, Lipase, Mannosidase, Oxidoreduktasen, pektinolytischem Enzym, Peptidoglutaminase, Peroxidase, Phytase, Polyphenoloxidase, proteolytischem Enzym, Ribonuklease, Transglutaminase oder Xylanase.

9. Verfahren nach Anspruch 8, wobei das pektinolytische Enzym ausgewählt ist aus der Gruppe bestehend aus Pektintranseliminase, Polygalactutonase, Pektinesterase.

10. Verfahren nach Anspruch 9, wobei die Cellulase eine Mehrkomponenten-Cellulasezubereitung oder eine Endoglucanase, bevorzugt eine *Humicola-*Endoglucanase, besonders eine *Humicola insolens*-Endoglucanase, oder eine Endoglucanase, bevorzugt eine *Thielavia*-Endoglucanase, bevorzugt eine *Thielavia terrestris*-Endoglucanase, oder eine Variante davon ist.

11. Verfahren nach Anspruch 10, wobei das proteolytische Enzym eine Protease ist, bevorzugt eine Serinprotease, insbesondere abgeleitet aus einem Stamm von *Bacillus,* wie *Bacillus lentus* oder *Bacillus clausii,* oder eine Variante davon.

12. Verfahren nach einem beliebigen der Ansprüche 1-11, wobei weiterhin ein oder mehrere Mittel ausgewählt aus der Gruppe bestehend aus Dispersionsmitteln, oberflächenaktiven Mitteln, antimikrobiellen Mitteln und Bioziden vorhanden sind.

13. Verfahren nach einem beliebigen der Ansprüche 1-12, wobei die Oberfläche eine harte, weiche oder poröse Oberfläche ist.

14. Verfahren nach Anspruch 13, wobei die Oberfläche eine Membran ist.

15. Verfahren nach einem beliebigen der Ansprüche 1-14, wobei die Biofilmentfernung bei einer Temperatur von zwischen 10-70°C, bevorzugt 40-60°C durchgeführt wird.

16. Verwendung einer alpha-Amylase wie in einem beliebigen der Ansprüche 1-15 definiert zur Verhinderung oder Entfernung von Biofilm von Oberflächen.

17. Verwendung nach Anspruch 16, wobei weiterhin Enzyme und/oder Mittel wie in Ansprüchen 1-15 definiert verwendet werden.

## Revendications

1. Méthode de prévention, d'élimination, de réduction ou de perturbation d'un biofilm présent sur une surface, comprenant la mise en contact de la surface avec une alpha-amylase ayant la séquence représentée par SEQ ID NO : 4, ou une alpha-amylase présentant au moins 90 % d'identité avec SEQ ID NO : 4.

2. Méthode selon la revendication 1, dans laquelle l'alpha-amylase est dérivée d'une souche de *Bacillus.*

3. Méthode selon les revendications 1 ou 2, dans laquelle la surface contaminée ou susceptible d'être contaminée par un biofilm est mise en contact entre 1 minute et 2 jours.

4. Méthode selon l'une quelconque des revendications 1 à 3, dans laquelle un tensioactif est en outre présent.

5. Méthode selon l'une quelconque des revendications 1 à 4, dans laquelle l'alpha-amylase comprend Asn-Gly-Thr-Met-Met-Gln-Tyr-Phe-Glu-Trp dans sa région d'acides aminés N-terminale.

6. Méthode selon l'une quelconque des revendications 1 à 5, dans laquelle l'alpha-amylase est utilisée dans une concentration entre 0,005 et 500 mg de protéine enzymatique, de préférence entre 0,01 et 100 mg de protéine enzymatique par 1 de solution de contrôle du biofilm.

7. Méthode selon l'une quelconque des revendications 1 à 6, dans laquelle l'alpha-amylase comporte un pourcentage (%) d'amidon hydrolysé qui est supérieur à 15, de préférence 25, spécialement 35, après 5 heures à 40 °C, 3 mg de protéine enzymatique par g d'amidon, pH 8,0.

8. Méthode selon l'une quelconque des revendications 1 à 7, dans laquelle en outre des enzymes sont présentes, lesquelles enzymes sont choisies dans le groupe constitué d'une aminopeptidase, amylase, carbohydrase, carboxypeptidase, catalase, cellulase, chitinase, cutinase, cyclodextrine glycosyltransférase, désoxyribonucléase, estérase, alpha-galactosidase, bêta-galactosidase, glucoamylase, alpha-glucosidase, bêta-glucosidase, halogénoperoxydase, invertase, laccase, lipase, mannosidase, oxydoréductases, enzyme pectinolytique, peptidoglutaminase, peroxydase, phytase, polyphénol-oxydase, enzyme protéolytique, ribonucléase, transglutaminase, ou xylanase.

9. Méthode selon la revendication 8, dans laquelle l'enzyme pectinolytique est choisie dans le groupe constitué de pectine transéliminase, polygalactutonase, pectine estérase.

10. Méthode selon la revendication 9, dans laquelle la cellulase est une préparation de cellulase à composants multiples ou une endoglucanase, de préférence une endoglucanase de *Humicola,* spécialement une endoglucanase de *Humicola insolens,* ou une endoglucanase, de préférence une endoglucanase de *Thielavia,* de préférence une endoglucanase de *Thielavia terrestris,* ou l'un de ses variants.

11. Méthode selon la revendication 10, dans laquelle l'enzyme protéolytique est une protéase, de préférence une sérine-protéase, spécialement dérivée d'une souche de *Bacillus,* comme *Bacillus lentus* ou *Bacillus clausii,* ou l'un de leurs variants.

12. Méthode selon l'une quelconque des revendications 1 à 11, dans laquelle en outre un ou plusieurs agents choisis dans le groupe constitué d'agents de dispersion, de tensioactifs, d'antimicrobiens, et de biocides, sont présents.

13. Méthode selon l'une quelconque des revendications 1 à 12, dans laquelle la surface est une surface dure, mole, ou poreuse.

14. Méthode selon la revendication 13, dans laquelle la surface est une membrane.

15. Méthode selon l'une quelconque des revendications 1 à 14, dans laquelle l'élimination du biofilm est réalisée à une température entre 10 et 70 °C, de préférence entre 40 et 60 °C.

16. Utilisation d'une alpha-amylase telle que définie dans l'une quelconque des revendications 1 à 15 pour la prévention ou l'élimination d'un biofilm de surfaces.

17. Utilisation selon la revendication 16, dans laquelle d'autres enzymes et/ou agents tels que définis dans les revendications 1 à 15 sont utilisés.
